# EUROPÄISCHE PATENTSCHRIFT

(11) **EP 0 484 581 B1**
(45) Veröffentlichungstag und Bekanntmachung des Hinweises auf die Patenterteilung: **15.06.1994**
(21) Anmeldenummer: 90121343.9
(22) Anmeldetag: 08.11.1990
(51) Int. Cl.: A61K 31/19, A61K 31/00, A61K 31/557

(54) **Verwendung von Thromboxan-A2-Antagonisten zur Verhinderung von degenerativen Vorgängen im penilen Gewebe**
Use of thromboxane A2 receptor antagonists for preventing degenerative processes in penile tissues
Utilisation des antagonistes du thromboxanne-A2 pour prévenir les processus dégénératifs des tissus pénils

(43) Veröffentlichungstag der Anmeldung: 13.05.1992
(73) Patentinhaber: BOEHRINGER MANNHEIM GMBH, 68298 Mannheim (DE)
(72) Erfinder: Pill, Johannes Dr.rer.nat.Dr.med., W-6906 Leimen 3 (DE); Jünemann, Klaus-Peter Dr., W-6800 Mannheim 1 (DE); Aufenanger, Johannes Dr., W-6945 Hirschberg 2 (DE); Konrad, Thomas Dr., W-6903 Neckargemünd/Dilsberg (DE)

(56) Entgegenhaltungen:
- Journal of Urology, Band 141, Nr. 1, Januar 1989, Seiten 182-186, The Williams & Wilkins Co.; H. Hedlund et al.: "Characterization of contraction-mediating prostanoi8d receptors in human penile erectile tissues"
- Circulation, Band 81, (1 Ergänz 1), Januar 1990, Seiten I-69 - I-78; G.I. Fiddler et al.: "Premliminary clinical studies with thromboxane systhase inhibitors and thromboxane receptor blockers a review"
- Archives of Andrology, Band 17, 1986, Seiten 233-234, Hemisphere Publishing Corp.; M.S.Bornman et al.: "Plateletes and thromoxane A2 in the pathogenesis of aging penile vascular changes and impotence"
- Archives of Andrology, Band 20, Seiten 101-102, Homisphere Publishing Corp.; J. Backon: "Possible utility of a thromboxane syntehtase inhibitor in preventing penile vascular changes and impotence during aging"
- SEMINARS IN UROLOGY, BAND VIII, Nr. 2, May 1990, K.P. JÜNEMANN et alpages 80-93

## Beschreibung

Die vorliegende Erfindung betrifft die Verwendung von Thromboxan (TX)-A₂-Rezeptor-Antagonisten zur Herstellung von Arzneimitteln zur Verhinderung von degenerativen Veränderungen bei glatten Muskelzellen im penilen Gewebe.

Eicosanoide wie Prostaglandine, Thromboxane und Leukotriene sind eine Gruppe von körpereigenen Stoffen, die an physiolgischen Regelmechanismen beteiligt sind. In diesem Zusammenhang sind diese Stoffe von Bedeutung für die lokale Regulation. Sie wirken dabei sowohl direkt auf das Erfolgsorgan als auch indirekt über andere Hormone und Überträgerstoffe. Ihre besondere Bedeutung scheint die eines membranständigen Schutzmechanismus zu sein, der bei Störung der zellulären Integrität aktiviert wird und gemeinsam mit anderen Faktoren das Gewebe an die Belastungssituation anpaßt. Dagegen können erhöhte lokale Konzentrationen dieser Substanzen, z.B. bei Gewebsschädigung oder -zerstörung, zusätzlich zu einer Aggravitation des Krankheitsgeschehens beitragen (K. Schrör, Prostaglandine, Thieme-Verlag, Stuttgart 1983; B.P. Curtis-Prior, Prostaglandins, Churchill Livingstone, Edinburgh, 1988). Das besondere pharmakologische Interesse besteht darin, durch selektive Eingriffe in diese Pathomechanismen einer Schädigung des Organismus im weitesten Sinne oder einer Krankheit vorzubeugen oder diese zu behandeln.

Bei erektiler Insuffizienz wurden ultrastrukturelle Veränderungen des penilen Gewebes gefunden. Als ein charakteristisches pathologisches Zeichen ist ein Verlust des kontraktilen Myofilamente Actin und Myosin beschrieben (Diabetologica 25, 424, 1983). Eine Aggregation von Mitochondrien ist vor allem im Frühstadium dieser Veränderung festzustellen (Ophtalmologica 191, 172, 1985). Beim Fortschreiten dieser degenerativen Prozesse kommt es zu weiteren intrazellulären Veränderungen, wie Anstieg der perinukleären Organellen und zur Zellkerndegeneration (Seminars in Urology, VIII (2) 80-93, 1990). Diese pathologischen Vorgänge enden schließlich in einer Schwellkörperatrophie (Proceedings of the Third Biennial World Meeting on Impotence, Boston, MA, October 6-9, 1988; Verhandlungsbericht DGU 40 Tagung, Saarbrücken September 28-October 1, 1988, Berlin, Springer Verlag, 357, 1989). Die morphologischen Veränderungen korrelieren mit dem klinischen Bild erektiler Störungen. Dies legt nahe, daß die beschriebenen degenerativen Veränderungen und nicht die penilen Arterien eine wesentliche Rolle in der Pathologie der organisch bedingten Erektionsstörungen spielen (Seminars in Urology, VIII (2) 80-93, 1990). In einer weiteren Untersuchung wurde ein Zusammenhang zwischen einem gestörten Lipidstoffwechsel und der vaskulären erektilen Dysfunktion diskutiert (Int. J. Impotence Res., September 1990, 2, Suppl. 2, 34).

Wesentliche Veränderungen bei der Degeneration des Penisgewebes, die eine verschiedengradige Insuffizienz des erektilen Gewebes bedingen, sind die Schwellkörperatrophie, Zellkerndegeneration und der Myofilamentabbau. Wirkstoffe, die diese Prozesse verhindern oder stark einschränken, sind bei einer Reihe von pathologischen Zuständen von therapeutischem Wert.

Bisher wird angenommen, daß Thromboxan A₂ für Impotenz verantwortlich ist, die durch einen reduzierten Bluteinstrom aufgrund pathologisch veränderter peniler Gefäße verursacht wird (Archives of Andrology 17 (1986), S. 233). In diesem Zusammenhang werden in der Arbeit von Fiddler et al., Circulation Vol. 81, No. 1 (1990), 1. Ergänzung, S. 1-69 bis 1-78 Thromboxan-Synthetase-Inhibitoren und Thromboxan-Rezeptor-Blocker hinsichtlich ihres Effektes auf die Blutplättchenaggregation untersucht. Auch die Arbeit von Backon in Archives of Andrology 20, (1988), S. 101 diskutiert den möglichen Einsatz eines Thromboxan-Synthetase-Inhibitors zur Prävention peniler vaskulärer Veränderungen.

In "The Journal of Urology", Vol. 141(1), January 1989, S. 182 - 186 wird der kontraktionshemmende Effekt des TxA₂-Rezeptor-Antagonisten L-636.499 auf die glatte Schwellkörpermuskulatur beschrieben.

Der vorliegenden Erfindung lag die Aufgabe zugrunde, geeignete Arzneimittel zur Verfügung zu stellen, die zur Behandlung von degenerativen Veränderungen der glatten Muskelzellen des Penisgewebes verwendet werden können.

Im Rahmen der vorliegenden Erfindung wurde nun überraschenderweise gefunden, daß TXA₂-Rezeptor-Antagonisten degenerative Veränderungen der glatten Muskelzellen im Penis, die zu einer Insuffizienz des erektilen Gewebes führen, herabsetzen.

Thromboxan(TX)-A₂-Rezeptor-Antagonisten vermögen die beim Kaninchen durch spezielle Diäten eintretenden degenerativen Veränderungen im Gewebe des Penis zu reduzieren. Die Substanzen eignen sich daher zur Behandlung und Verhinderung von degenerativen Vorgängen im Penisgewebe, die bei Vorliegen von Risikofaktoren, wie Medikamenteneinnahme, Nikotinabusus und Alkoholismus, häufig nachzuweisen sind.

Die TXA₂-Rezeptor-Antagonisten verhindern die Degeneration des penilen Gewebes in Dosen, welche in der gleichen Größenordnung liegen, wie diejenigen, die bei therapeutischem Einsatz von TXA₂-Rezeptor-Antagonisten üblich sind. Es ist zu erwarten, daß die erfindungsgemäße Unterdrückung degenerativer Vorgänge im Penis und die damit verbundene Verhinderung der Insuffizienz des erektilen Gewebes mittels TXA₂-Rezeptor-Antagonisten bei einer Reihe von klinischen Situationen von therapeutischem Wert sein wird. Dazu gehören neben den oben erwähnten Risikogruppen die Prävention einer Penisprothese sowie die Prävention und/oder Reduktion der intravenösen Papaverininjektion.

Für den Zweck der vorliegenden Erfindung geeignete TXA₂-Rezeptor-Antagonisten sind Sulotroban (BM 13.177), Daltroban (BM 13.505), L-640.035, L-641.953, L-655.240, OKY-046, OKY-1581, ONO-1078, ONO-1270, ONO-3708, ONO-11113, SQ 28668, SQ 29548, SQ 30741, AH 23848, SK&F 88046, EP 045, S-145, Sch 37224, KF 4939, EG-626, N-Benzyltrimetoquinol-Analoga, 9,11-Epoxi-9-homo-14-thiaprost-5-ensäurederivate, 13-Azaprostansäure, 3-Alkylaminopinanderivate, Pinan-TXA₂-derivate, 9,11-Azo-12-oxa-15-hydroxyprostansäure, 9.11-Epoxyiminoprosta-5,11-diensäure und Dandrolen, die sämtlich aufgeführt sind in Cumulated Index Medicus 1978-1989, Subject Index unter Thromboxanes oder Thromboxane-A₂-Antagonists and Inhibitors, sowie R 68070, AA 2414, SQ 33261, SQ 33552, FR 106881, BayU 3405, ICI 192605, L 670596, L 657925, L 657926, GR 32191 alle genannt in Thrombosis and Haemostasis 62 (1) 1-647 (1989) und W-4099, SCRIP Nr. 1537, 28 (1990). Insbesondere kommen als TXA₂-Rezeptor-Antagonisten solche Verbindungen in Frage, die in den Patentanmeldungen EP-A-31,954, EP-A-221,344, EP-A-223,593, EP-A-239,907, EP-A-304,271, EP-A-322,692, EP-A-325,245, EP-A-353,448, EP-A-356,989, EP-A-361,113, EP-A-365-183 oder DE-P 4006640.1 beschrieben sind.

Die Untersuchungen, die für sämtliche TXA₂-Antagonisten als repräsentativ angesehen werden können, wurden mit Daltroban (4-[2-(4-Chlorphenyl)-sulfonylaminoethyl]phenylessigsäure) durchgeführt.

Diese Verbindung ist in der EP-B-31,954 beschrieben. Zur Verhinderung degenerativer Vorgänge im penilen Gewebe werden TXA₂-Antagonisten systemisch vorzugsweise enteral, besonders bevorzugt oral aber auch parenteral (i.v.) verabreicht. Die Dosis variiert gemäß den Bedürfnissen des einzelnen Patienten, und kann vom behandelnden Arzt bestimmt werden. Für Daltroban dürfte im allgemeinen eine tägliche Dosis von etwa 10-1000 mg, insbesondere 100-800 mg verwendet werden. Der Dosisbereich von 100-800 mg pro Tag entspricht demjenigen, der bevorzugt therapeutisch bei Thromboxanbedingten Erkrankungen eingesetzt wird. Die tägliche Dosis kann durch einmalige Gabe oder mehrmals täglich durch Gabe der entsprechenden Teilmengen verabreicht werden. Die anderen TXA₂-Rezeptor-Antagonisten können in Übereinstimmung mit der jeweiligen Dosierungsvorschrift, im einzelnen festgelegt durch den behandelnden Arzt, verabreicht werden.

Wegen der Möglichkeit der oralen Verabreichung der TXA₂-Rezeptor-Antagonisten ist die Behandlungsdauer zeitlich nicht begrenzt, was für die erfindungsgemäße Verwendung wesentlich ist.

Als Verabreichungsform kommen die für die systemische Verabreichung üblichen festen oder flüssigen Darreichungsformen in Betracht, wie z.B. Suppositorien und feste orale Darreichungsformen, wie Kapseln, Tabletten, Lacktabletten, Dragees, Pillen, Granulate und dergleichen, oder flüssige orale Darreichungsformen, wie Lösungen, Sirupe, Suspensionen, Elixiere und dergleichen oder parenterale Darreichungsformen, wie Infusions- oder Injektionslösungen, die intravenös oder intramuskulär injiziert werden können.

Es liegt im Bereich der vorliegenden Erfindung, die TXA₂-Rezeptor-Antagonisten in jeder für die Verabreichung geeigneten Menge in die enterale oder orale Darreichungsform einzuarbeiten. Es ist jedoch bevorzugt, Präparate herzustellen, die pro Dosierungseinheit den Wirkstoff in einer Menge bis zu 800 mg, vorzugsweise von etwa 100 oder 200 mg enthalten. Besonders bevorzugt ist die Herstellung von Kapseln, Tabletten und Lacktabletten als Dosierungseinheit. Diese können nach den jeweiligen vom Arzt festzulegenden Erfordernissen ein- oder mehrmals täglich appliziert werden.

Die Herstellung der oben genannten Gebrauchsformen kann in üblicher Weise, z.B. anhand des nachstehenden Beispiels für Daltroban erfolgen.

### Beispiel 1 Herstellung einer Tablettier- oder Kapselfüllmasse

| Tabletten mit 200 mg Daltroban | | |
|---|---|---|
| Zusammensetzung | | mg/Kapsel |
| 1. | Daltroban, fein | 200.0 |
| 2. | Lactose x 1H₂O | 50.0 |
| 3. | Crospovidon | 10.0 |
| 4. | Povidon 25.000 | 10.5 |
| 5. | Crospovidon | 5.0 |
| 6. | Siliziumdioxyd kolloidal | 2.5 |
| 7. | Zellulose mikrokristallin | 20.0 |
| 8. | Magnesiumstearat | 5.0 |
| | Füllgewicht | 303.0 |
| 9. | Gelatinekapseln | Größe 0 |

Die Bestandteile werden miteinander nach herkömmlichen Verfahren gemischt und feucht oder trocken granuliert. Die fertige Masse kann zu Kernen verpreßt und direkt als Tabletten oder mit einem Film überzogen als Filmtabletten verwendet werden. Die Masse kann auch direkt in Kapseln, wie z. B. Gelatinekapseln, abgefüllt werden.

### Beispiel 2 Therapeutische Wirkung

Die therapeutische Wirkung von Daltroban bei degenerativen Veränderungen des penilen Gewebes kann dem nachfolgenden Versuch entnommen werden.
a) Allgemeines Prinzip
   Bei der Verabreichung spezieller Diäten an Kaninchen kommt es im Penis entsprechend den Vorgängen bei Menschen (Investigative Urology 3 53-59, 1989; Seminars in Urology VIII (2) 80-93, 1990) zu einer Zellkerndegeneration, einem intrazellulären Verlust des Myofilaments und einer Schwellkörperatrophie. Diese Vorgänge führen zu einer verschiedengradigen Insuffizienz des erektilen Gewebes. Die Diät wurde über 96 Tage verabreicht. Daltroban wurde in einer Dosierung von 10 mg/kg/d ab dem 42. Tag zusätzlich verabreicht. Die Studie gibt Aufschluß, ob Daltroban eine Wirkung auf die degenerativen Prozesse im penilen Gewebe besitzt. Das gewählte Versuchsdesign stellt möglicherweise gegenüber der Daltroban-Verabreichung während der gesamten Behandlungsdauer erschwerte Testbedingungen dar, kommt aber im Hinblick auf eine therapeutische Anwendung der Situation beim Menschen am nächsten. Das Ausmaß der degenerativen Veränderungen wird anschließend an Transversalschnitten des Penis aufgrund licht- und elektronischen mikroskopischer Begutachtung bestimmt.
b) Versuchsbeschreibung
   Männliche weiße Neuseeland Kaninchen mit einem Körpergewicht von etwa 2,0 kg bei Versuchsbeginn erhielten über 96 Tage eine semisynthetische Diät, bestehend aus Kasein, Glukose/Stärke, Zellulose, 0.5% Cholesterin sowie der üblichen Salz- und Vitaminmischung. Daltroban wurde in einer Dosierung von 10 mg/kg/d ab dem 42. Tag zusätzlich verabreicht. Die Applikation erfolgte über das Trinkwasser und wurde über den Wasserverbrauch kontrolliert. Bei Versuchsende wurde aus dem apikalen, medialen und basalen Teil des Penis Segmente entnommen. Für die morphologische Begutachtung wurde die Penissegmente fixiert, dehydriert und schließlich eingebettet. Das Ausmaß der degenerativen Veränderungen wurde mit Hilfe licht- und transmissionselektronenmikroskopischer Begutachtung an Transversalschnitten der Penis-Segmente ermittelt. Folgende Charakteristika wurden für die Beurteilung herangezogen:
   1. Myofilamentabbau
   2. Zellkerndegeneration
   3. Schwellkörperatrophie
c) Ergebnisse
   Die ab dem 42. Tag zusätzlich mit Daltroban behandelten Tiere zeigen in den untersuchten Penissegmenten eine deutlich geringere Ausprägung des Myofilamentabbaus, der Zellkerndegeneration und der Schwellkörperatrophie im Vergleich zu den Diätkontrolltieren. Mit Standard-Diät gefütterte Kaninchen weisen keine degenerativen Veränderungen auf.

Die morphologisch eindeutig und in weiteren Untersuchungen reproduzierbar festgestellte Reduktion der degenerativen Veränderungen des penilen Gewebes durch Daltroban zeigt, daß die Substanz eine Hemmwirkung auf degenerative Prozesse des penilen Gewebes besitzt. Die Ergebnisse belegen eine neue Wirkqualität der Substanz.

Hiermit ist anhand von Daltroban eindeutig gezeigt, daß TXA₂-Rezeptor-Antagonisten die Zunahme degenerativer Veränderungen des Penis reduzieren.

Die hier beschriebene neue Verwendung von TXA₂-Rezeptor-Antagonisten kann sowohl bei Stoffwechselgesunden als auch Stoffwechselkranken, wie zum Beispiel Dyslipidämiker und Diabetiker, bei Normotonikern und auch Hypertoniker angezeigt sein, wenn die oben beschriebenen degenerativen Prozesse eintreten. Im besonderen sei auf eine Zusatzbehandlung mit Daltroban bei Medikation mit bekanntem Auftreten von Potenzstörung hingewiesen.

## Patentansprüche

1. Verwendung von TXA₂-Rezeptor-Antagonisten zur Herstellung von Arzneimitteln zur Verhinderung von degenerativen Vorgängen bei glatten Muskelzellen im penilen Gewebe.

2. Verwendung gemäß Anspruch 1 zur Herstellung von Arzneimitteln zur Verhinderung der Schwellkörperatrophie.

3. Verwendung gemäß Anspruch 1 zur Herstellung von Arzneimitteln zur Verhinderung der Zellkerndegeneration.

4. Verwendung gemäß einem der Ansprüche 1 zur Herstellung von Arzneimittel zur Verhinderung des Abbaus der Myofilamente.

5. Verwendung gemäß einem der Ansprüche 1-4, dadurch gekennzeichnet, daß es sich bei den degenerativen Vorgängen um physikalisch, chemisch, chirurgisch oder pathologisch bedingte Veränderungen handelt.

6. Verwendung gemäß einem der Ansprüche 1-5 bei Nikotinabusus und Alkoholismus.

7. Verwendung von TXA₂-Rezeptor-Antagonisten gemäß Anspruch 1-6, dadurch gekennzeichnet, daß als TXA₂-Rezeptor-Antagonist Daltroban eingesetzt wird.

8. Verfahren zur Herstellung eines Arzneimittels, wobei ein als Wirkstoff eingesetzter TXA₂-Rezeptor-Antagonist gegebenenfalls zusammen mit einem pharmazeutisch geeigneten Trägermaterial und sonstigen Hilfsstoffen in eine für therapeutische Zwecke anwendbare Form gebracht wird, dadurch gekennzeichnet, daß man ein Heilmittel, wie es in einem der Ansprüche 1-7 beschrieben ist, herstellt, bestehend aus der Kombination von einem TXA₂-Rezeptor-Antagonist oder einem einen TXA₂-Rezeptor-Antagonisten enthaltenden Präparat zusammen mit der Information, welche im Zusammenhang mit der Behandlung steht, wie sie in einem der Anspüche 1-7 beschrieben ist.

## Claims

1. Use of TXA₂ receptor antagonists for the preparation of medicaments for the prevention of degenerative changes in smooth muscle cells in the penile tissue.

2. Use according to claim 1 for the preparation of medicaments for the prevention of erectile tissue atrophy.

3. Use according to claim 1 for the preparation of medicaments for the prevention of cell nucleus degeneration.

4. Use according to claim 1 for the preparation of medicaments for the prevention of the breakdown of the myofilaments.

5. Use according to one of claims 1-4, characterised in that, in the case of the degenerative processes, it is a question of physically, chemically, surgically or pathologically caused changes.

6. Use according to one of claims 1-5 in the case of nicotine abuse and alcoholism.

7. Use of TXA₂ receptor antagonists according to claims 1-6, characterised in that daltroban is used as TXA₂ receptor antagonist.

8. Process for the preparation of a medicament, whereby a TXA₂ receptor antagonist used as active material, possibly together with a pharmaceutically suitable carrier material and other adjuvants, is brought into a form usable for therapeutic purposes, characterised in that one prepares a curative agent as described in one of claims 1-7, consisting of a combination of a TXA₂ receptor antagonist or of a preparation containing a TXA₂ receptor antagonist, together with the information which stands in conjunction with the treatment as it is described in one of claims 1-7.

## Revendications

1. Utilisation d'antagonistes du récepteur du TXA₂, pour la fabrication de médicaments destinés à éviter les processus dégénératifs des muscles lisses du tissu pénien.

2. Utilisation d'antagonistes selon la revendication 1, pour la fabrication de médicaments destinés à éviter l'atrophie des corps caverneux.

3. Utilisation d'antagonistes selon la revendication 1, pour la fabrication de médicaments destinés à éviter la dégénérescence des noyaux cellulaires.

4. Utilisation d'antagonistes selon la revendication 1, pour la fabrication de médicaments destinés à éviter la destruction des myofilaments.

5. Utilisation d'antagonistes selon les revendications 1-4, caractérisée par le fait que les processus dégénératifs concernent des modifications provoquées par des phénomènes physiques, chimiques, chirurgicaux ou pathologiques.

6. Utilisation d'antagonistes selon l'une des revendications 1-5, dans des cas de tabagisme excessif ou d'alcoolisme.

7. Utilisation des antagonistes du récepteur du TXA₂, selon les revendications 1-6, caractérisée par l'emploi du daltroban comme antagoniste du récepteur du TXA₂.

8. Procédé de fabrication d'un médicament , dans lequel on utilise un antagoniste du récepteur du TXA₂ comme substance active avec, éventuellement, un véhicule pharmaceutique approprié et d'autres additifs sous une forme adaptée à des fins thérapeutiques et caractérisé en ce que l'on fabrique un médicament tel que décrit dans l'une des revendications 1-7, comprenant un antagoniste du récepteur du TXA₂ ou une préparation contenant un antagoniste du récepteur du TXA₂, accompagnée de l'information concernant l'utilisation telle que décrite dans l'une des revendications 1-7.
